# EUROPÄISCHE PATENTANMELDUNG

(11) **EP 1 522 595 A2**
(43) Veröffentlichungstag der Anmeldung: **13.04.2005**
(21) Anmeldenummer: 04017805.5
(22) Anmeldetag: 28.07.2004
(51) Int. Cl.: C12Q 1/68

(54) **Oligonukleotide, Verfahren und System zur Detektion von antibiotikaresistenzvermittelnden Genen in Mikroorganismen mittels der Echtzeit-PCR**

(30) Priorität: 28.08.2003 DE 10339609
(71) Anmelder: Forschungszentrum Karlsruhe GmbH, 76133 Karlsruhe (DE)
(72) Erfinder: Obst, Ursula, Dr., 76131 Karlsruhe (DE); Volkmann, Holger, 76227 Karlsruhe (DE); Schwartz, Thomas, Dr., 76131 Karlsruhe (DE); Kirchen, Silke, 76133 Karlsruhe (DE)
(74) Vertreter: Fitzner, Uwe, Dr.

(57) **Zusammenfassung**

Oligonukleotid, Verfahren und System zur Detektion von Antibiotikaresistenzvermittelnden Genen in Mikroorganismen mittels der Echtzeit-PCR umfassend:
- Verwendung einer ersten Primer-Nukleotidsequenz (A), die aus der Gruppe der Sequenzen bestehend aus den SEQ # 1 - 4 ausgewählt wird,
- Verwendung einer zweiten Primer-Nukleotidsequenz (B), die aus der Gruppe der Sequenzen bestehend aus den SEQ # 5 - 8 ausgewählt wird, wobei die Sequenzen 1 und 5, 2 und 6, 3 und 7, 4 und 8 als Primerpaare eingesetzt werden, und
- Verwendung mindestens eines ersten Farbstoffs (C) zum Nachweis der mittels der PCR amplifizierten DNA,
sowie deren Verwendung, insbesondere auf einem Biochip.

SEQUENCE LISTING

<110>  Forschungszentrum Karlsruhe GmbH

<120>  Oligonukleotid, Verfahren und System zur Detektion von
Antibiotikaresistenz-vermittelnden Genen in Mikroorganismen mittels der
Echtzeit-PCR

<130>  KFK0207DE

<160>  20

<170>  PatentIn version 3.1

<210>  1

<211>  27

<212>  DNA

<213>  Aritificial

<400>  1
caacactacc cggaagcaca gttcgtc                                    27

<210>  2

<211>  25

<212>  DNA

<213>  Aritificial

<400>  2
cctatggcgt gaaaaccaac gtgca                                          25

<210>  3

<211>  32

<212>  DNA

<213>  Aritificial

<400>  3
aatgacgcta tgatcccaat ctaacttcca ca                                  32

<210>  4

<211>  26

<212>  DNA

<213>  Aritificial

<400>  4
caactaacgc ggcactgttt cccaat                                         26

<210>  5

<211>  20

<212>  DNA

<213>  Aritificial

<400>  5
agagtttgat cmtggctcag                                                    20

<210>  6

<211>  17

<212>  DNA

<213>  Aritificial

<400>  6
attaccgcgg ctgctgg                                                       17

<210>  7

<211>  20

<212>  DNA

<213>  Aritificial

<400>  7
tctgcaatag agatagccgc                                                    20

<210>  8

<211>  20

<212>  DNA

<213>  Aritificial

<400>  8
ggagtagcta tcccagcatt                                                    20

<210>  9

<211>  20

<212>  DNA

<213>  Aritificial

<400>  9
ttctatcaam actggcarcc                                                    20

<210>  10

<211>  20

<212>  DNA

<213>  Aritificial

<400>  10
ccygttttat gtacccayga                                                    20

<210>  11

<211>  22

<212>  DNA

<213>  Aritificial

<400>  11
aaaatcgatg gtaaaggttg gc                                                22

<210>  12

<211>  19

<212>  DNA

<213>  Aritificial

<400>  12
gggaatgctg gatgcacaa                                                    19

<210>  13

<211>  22

<212>  DNA

<213>  Aritificial

<400>  13
agttctgcag taccggattt gc                                                22

<210>  14

<211>  26

<212>  DNA

<213>  Aritificial

<400>  14
cgcaacgttc aatttaattt tgttaa                                          26

<210>  15

<211>  19

<212>  DNA

<213>  Aritificial

<400>  15
ctgtgaggtc ggttgtgcg                                                  19

<210>  16

<211>  14

<212>  DNA

<213>  Aritificial

<400>  16
gccgtgcccc ggaa                                                       14

<210>  17

<211>  21

<212>  DNA

<213>  Aritificial

<400>  17
catgacccag ttcgccatat c                                                21

<210>  18

<211>  21

<212>  DNA

<213>  Aritificial

<400>  18
tggtctttct gcattcctgg a                                                21

<210>  19

<211>  16

<212>  DNA

<213>  Aritificial

<400>  19
tttggtccac dcgcca                                                      16

<210>  20

<211>  19

<212>  DNA

<213>  Aritificial

<400>  20
cctccattga gcggattca                                                    19

## Beschreibung

Die Erfindung betrifft neue Oligonukleotide sowie Verfahren und Systeme zur Detektion von Antibiotikaresistenz-vermittelnden Genen in Mikroorganismen mittels der Echtzeit-PCR unter Verwendung der neuen Oligonukleotide.

Antibiotika spielen in Bezug auf den Einfluss von Xenobiotika auf die Umwelt eine zunehmende Rolle. Durch zu häufige und gegebenenfalls falsche Anwendung als Therapeutikum, aber auch in Futtermittel zur Wachstumsföderung von Mastvieh werden Antibiotika durch den Menschen vermehrt in die Umwelt eingetragen. Sie können dabei über eine Vielzahl von Eintragspfaden aus antropogenen Quellen in die Umwelt gelangen und bewirken dort eine Anreicherung antibiotikaresistenter Bakterien. Besonders Bakterien mit mehrfachen Resistenzen können dann bei Infektionen von Mensch und Tier nur schwer bekämpft werden. Selbst hochwirksame Antibiotika, die daher heute nur als Reserveantibiotika, d. h. beim Versagen aller anderen eingesetzt werden, können ihre Wirkung durch Resistenzbildung verlieren.

Die Detektion von Antibiotika in der Umwelt, um das Ausmaß des unnatürlichen Eintrags zu bestimmen, ist jedoch häufig schwierig und erfordert eine teure analytische Ausrüstung. Dabei hinaus sind chemische Analysen nicht der Lage, Aussagen über die Wirkung der analysierten Substanz auf Organismen zutreffen.

Der Einfluss von Antibiotika auf Bakterienpopulationen kann jedoch direkt durch eine Zunahme resistenter Bakterien nachgewiesen werden und zeigt eine Gefährdung durch deren Ausbreitung in der Umwelt an.

Üblicherweise werden antibiotikaresistente Bakterien in Kultivierungsversuchen identifiziert, ohne die Resistenz-vermittelnden Gene direkt nachzuweisen. Solche klassischen mikrobiellen Verfahren mittels Antibiogramme sind langwierig, beschränken sich auf die Erfassung kultivierbarer Bakterien und erlauben keine Aussagen über die genetischen Ursachen der Resistenzen. Zudem sind relativ große Mengen der Bakterien dazu notwendig. Bisher verwendete molekularbiologische Verfahren wie herkömmliche PCR-Assays sind nicht quantifizierbar.

Die Polymerase Kettenreaktion (PCR) ist ein übliches und dem Fachmann bekanntes molekularbiologisches Verfahren zur Vermehrung (Amplifikation) weniger Mol einer beliebigen genomischen DNA-Sequenz in kürzester Zeit in vitro um Faktoren von 10⁶ bis 10⁸ zu vermehren (vgl. Römpp Lexikon Biotechnologie und Gentechnik, 2. Aufl., Thieme Verlag Stuttgart 1999, "Polymerase chain reaktion" Seite 627).

Die davon abgeleitete Echtzeit-PCR (real-time PCR) erlaubt die Analyse (Quantifizierung) der Amplifikation durch Detektion einer Fluoreszenz von einem Farbstoff die entweder direkt oder indirekt mit der Vermehrung der amplifizierten DNA assoziiert ist (vgl. Übersicht in Journal of Molecular Endocrinology 2000 Vol. 25, S. 169 - 193).

Aus George E. Killgore et al., Journal of Clinical Microbiology, Juli 200, Seiten 2516 - 2519 "A 5' Nuclease PCR (TaqMan) High-Throughput Assay for Detection of the *mecA* Gene in Staphylococci" ist bekannt, zur schnellen Untersuchung einer großen Menge von Krankenhauspatienten auf das Vorhandensein des *mecA* Gens, welches in Staphylokokken für die Resistenz gegenüber dem Antibiotikum Methicillin verantwortlich ist, die Echtzeit-PCR mittels des TaqMan Verfahrens (P. M. Holland et al. in Proc. Natl. Acad. Sci. USA 88:7276-7280, 1991) anzuwenden.

Allerdings sind die dort verwendeten Primer und die Sonde für das *mecA* Gen nicht für die Anwendung auf Biochips geeignet, da die verwendeten Primer nicht multiplex fähig, d. h. sie sind zu lang, um eine schnelle und gleichmäßige Kinetik zu erreichen. Ferner werden nur Primer und eine Sonde für das *mecA* Gen offenbart. Weitere Antibiotikaresistenzgene, entsprechende Primer oder Sonden sind nicht erwähnt und werden auch nicht gemeinsam verwendet.

Damit aber Primer und ggf. Sonden für mehrere Gene gleichzeitig auf Biochips eingesetzt werden können, müssen diese sich in ihren kinetischen Eigenschaften einander annähern, d. h. multiplexfähig sein. Ansonsten würden verfälschte Ergebnisse für ein bestimmtes Gen erhalten werden, wenn das zugehörige Primerpaar z. B. eine günstigere Kinetik aufweist als die übrigen.

Aufgabe der vorliegenden Erfindung ist es, sichere und schnelle Systeme zum Nachweis und zur Quantifizierung klinisch relevanter antibiotikaresistenter Bakterien in der Umwelt mittels molekularbiologischer Nachweissysteme bereitzustellen, die insbesondere auf Biochip-Technologie umsetzbar und zudem artspezifisch sind.

Demgemäß wurden die neuen Oligonukleotide umfassend eine Nukleotidsequenz ausgewählt aus der Gruppe der Sequenzen bestehend aus den SEQ # 1 - 8 gefunden, die sich als Primer für die PCR, insbesondere Echtzeit-PCR eignen.

Auch wurden neue Oligonukleotide umfassend eine Nukleotidsequenz ausgewählt aus der Gruppe der Sequenzen bestehend aus den SEQ # 9 - 12 gefunden, die sich als Sonden für die Echtzeit-PCR eignen.

Zusätzlich wurde das erfindungsgemäße Verfahren zur Detektion von Antibiotikaresistenz-vermittelnden Genen in Mikroorganismen mittels der Echtzeit-PCR umfassend:
- Einsatz mindestens eines ersten Oligonukleotids (A) gemäß Anspruch 1 oder 2 als Primer ,
   und
- Einsatz mindestens eines ersten Farbstoffs (C) zum Nachweis der mittels der PCR amplifizierten DNA,
gefunden, wobei insbesondere
- die erste Primer-Nukleotidsequenz (A), aus der Gruppe der Sequenzen bestehend aus den SEQ # 1 - 4 ausgewählt wird,
- eine zweite Primer-Nukleotidsequenz (B), aus der Gruppe der Sequenzen bestehend aus den SEQ # 5 - 8 ausgewählt wird, und
- die Sequenzen SEQ # 1 und 5, 2 und 6, 3 und 7, 4 und 8 als Primerpaare eingesetzt werden.

Mit dem erfindungsgemäßen Verfahren ist es möglich, Antibiotikaresistenz-vermittelnde Gene unter Echtzeit-Bedingungen und quantitativ art- und genspezifisch nachzuweisen. Mit anderen Worten, die Primer-Nukleotidsequenzen wurden so ausgewählt, das mit ihnen antibiotika- und artspezifische Tests zur Detektion von Antibiotikaresistenz-vermittelnden Genen in Mikroorganismen und Gesamt-DNA aus bakteriellen Populationen mit Hilfe der Echtzeit-PCR möglich wird.

Insbesondere sind die Primerpaare der Antibiotika-Nachweissysteme in ihrer Länge einander angeglichen, um eine PCR im multiplex-Ansatz zu erleichtern, d. h. mit dem erfindungsgemäßen Verfahren und den darin eingesetzten Primer-Nukleotidsequenzen sowie den unten beschriebenen Sonden ist es möglich gleichzeitig, also in einem "Topf" auf mehrere Antibiotikaresistenz-vermittelnde Gene hin zu untersuchen.

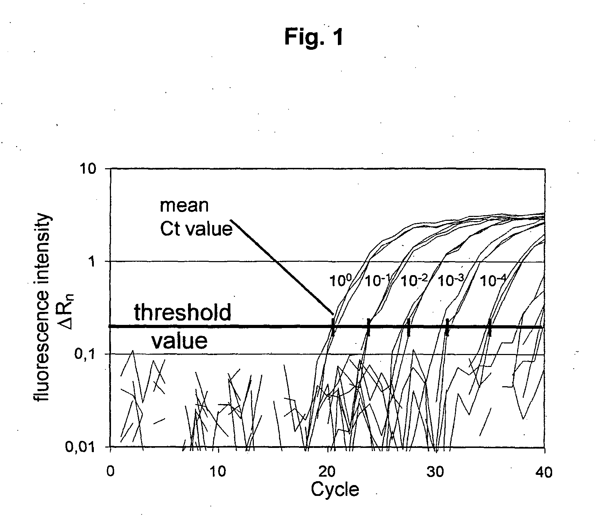

Die ersten Primer-Nukleotidsequenzen (A), die aus der Gruppe der Sequenzen bestehend aus den SEQ # 1 - 5 ausgewählt werden, sind die forward Primer. Die zweiten Primer-Nukleotidsequenzen (B), die aus der Gruppe der Sequenzen bestehend aus den SEQ # 6 - 10 ausgewählt werden, sind entsprechend die reverse Primer, wobei die Sequenzen 1 und 6, 2 und 7, 3 und 8, 4 und 9, 5 und 10 als Primerpaare eingesetzt werden. Die genauen Sequenzen gehen aus Fig. 1 hervor.

Die Mikroorganismen der Gattungen Pseudomonas, Enterobacteriaceae, Staphylococcus und Enterococcus sind besonders bevorzugt für die Untersuchungen des Einfluss des Menschen auf seine Umwelt, da sie besonders in wässrigen Umweltproben vorkommen. Fakultativ können diese pathogen sein. Besonders bevorzugt sind hiervon insbesondere *Pseudomonas aeruginosa*, *Enterobacter cloacae*, *Staphylococcus aureus* und *Enterococcus faecium*.

Diese Mikroorganismen dienen teilweise als Indikatorbakterien fäkaler Verunreinigung oder weisen auf unsachgemäße technische Aufbereitungsprozesse, z. B. in der Trinkwassertechnologie hin.

Mit der Zunahme der Häufigkeit der bakteriellen Resistenz spielt das Glykopeptid Vancomycin eine wichtige Rolle als Reserveantibiotikum für die Behandlung von Infektionen mit gram-positiven, resistenten Pathogenen. Jedoch sind Vancomycin resistente Enterokokken bereits in Fleisch, Hühnerfäkalien, Abwasser und sogar im Oberflächenwasser nachgewiesen worden. Das *van*A Gen als dominanter Resistenzfaktor in Enterokokken kodiert für eine Ligase, die in der Lage ist, die Zellwandeigenschaften zu Veränderungen und somit die Affinität für Vancomycin herabsetzt.

*Pseudomonas aeruginosa* ist gegebenenfalls pathogen und häufig mit nosokomialen Infektionen assoziiert. Insbesondere Arten, die ein *bla*_{VIM} Gen tragen zeigen eine Resistenz für β-Lactamase-stabile Antibiotika wie Imipenem. Abgesehen von dieser klinischen Relevanz, ist *Pseudomonas aeruginosa* auch in der Umwelt vorhandenen und wurde sogar im Trinkwasser aufgefunden.

Zur Zeit sind sieben Varianten des Imipinemresistenz vermittelnden Gens bla_{VIM} bekannt und sequenziert. 18 Imipenem resistente *Pseudomonas aeruginosa* Stämme wurden aus unterschiedlichen Abwehrsondenoberflächen gemessen isoliert. Sequenzierungen des bla_{VIM} Gens zeigten, dass ausschließlich das Gen bla_{VIM}-2 vorlag. Daher wurden Primer und Sonde auf eine spezifische Detektion von bla_{VIM}-2 ausgelegt.

Die Resistenzgene bla_{VIM} liegen auf Plasmiden codiert. Außer bei *Pseudomonas aeruginosa* wurden diese Gene auch auf Plasmiden anderer Bakterien gefunden. Gegenüber genomischen lokalisierten Genen unterliegen die auf Plasmiden liegenden Gene anderen Ausbreitungsmechanismen. DNA kann hier zwischen Bakterien gleicher und unterschiedlicher Spezies ausgetauscht werden (horizontaler Gentransfer). Beispielsweise können Resistenzgene gekoppelt mit weiteren plasmidgebundenen Genen für das Überleben des Bakteriums überaus positiv sein, während der fehlende Selektionsdruck durch das Antibiotikum sich negativ auf die Persistenz des Resistenzgens in der Zelle auswirken kann. Es ist bekannt, dass bei fehlendem Selektionsdruck Bakterien die entsprechenden Plasmide aus der Zelle ausschleusen können. Das Gen bla_{VIM} kann daher als Indikator für die Ausbreitung von Resistenzgenen, die auf solchen mobilen genetischen Elementen liegen, dienen.

Das enterobakterielle Gen ampC ist ein häufig induzierbares chromosomal codiertes Resistenzgen für die Synthese einer β-Lactamase, die in der Lage ist Penicillin G, Ceftazidim und weitere Breitbandcephalosporine zu hydrolisieren. *Enterobacter cloacae* mit dem *amp*C Resistenzgen kommen in Fäkalien und Abwasser vor.

Staphylokokken sind opportunistische Bakterien und treten häufig bei nokosomialen Infektionen auf. Fast 50 Prozent aller Infektionen, die bei der intensive Pflege auftreten, können *Staphylococcus aureus* oder coagulase negativen Staphylokokken (CNS) zugeschrieben werden. Seit der Verwendung des Antibiotikums Methicillin hat das Auftreten resistenter *Staphylococcus aureus* und CNS stark zugenommen, die das mecA-Gen tragen, welches für die Methicillinresistenz essenziell ist.

Besonders die Antibiotika Imipenem, Ampicilin, Methicillin, Vancomycin sind daher von Interesse, weil Bakterien mit diesbezüglichen Resistenzen vom klinischer Relevanz sind und gute Indikatoren für die Belastung aquatischer Systeme mit antibiotikaresistenter Bakterien sind.

Daher sind auch die Antibiotikaresistenz-vermittelnden Gene aus der Gruppe bestehend aus *bla*_{VIM}, *ampC, mec*A und *van*A die für die Resistenz in den entsprechenden Mikroorganismen verantwortlich sind, ebenfalls von besonderem Interesse und Ziele des erfindungsgemäßen Verfahrens.

Die von der PCR abgeleitete Echtzeit-PCR (real-time PCR) erlaubt die Analyse (Quantifizierung) der Amplifikation durch Detektion einer Fluoreszenz von einem Farbstoff die entweder direkt oder indirekt mit der Vermehrung der amplifizierten DNA assoziiert ist.

Eine direkte Methode verwendet einen Farbstoff, der unspezifisch an doppelsträngige DNA bindet und nur bei dieser Bindung fluoresziert. Bei der Amplifikation der Ziel-DNA während der Echtzeit-PCR bindet sich dieser Farbstoff an die neugebildete doppelsträngige DNA, so dass die messbare Fluoreszenz ansteigt.

Eine weitere direkte Methode ist der Einsatz von Fluoreszenz-Resonanz-Energie-Transfer Sonden (FRET), die an die amplifizierte DNA binden. Eine FRET-Sonde ist ein kurzes Oligonukleotid, welches komplementär zu einer der Ziel-Genomssequenzstränge ist. Die Sonde umfasst zwei fluoreszierende Farbstoffe, einen "Reporter" am 5' und einen "Quencher" am 3' Ende der Sonde. In den Sonden werden die Farbstoffe im ungebundenen Zustand durch eine Schleifenanordnung (Hairpin Loop) in räumlicher Nähe gehalten. Die Schleifenanordnung wird durch komplementäre Sequenzen an den Enden der eigentlichen Sondensequenz erzeugt. Der Quencher Farbstoff ist auf Grund seiner Nähe zu dem Reporter in der Lage, dessen Fluoreszenz durch die FRET zu "quenchen" d. h. auszulöschen. Diese Sonde, auch "Beacon" genannt, wird bei der Echtzeit-PCR Reaktion zusammen mit dem vorwärts und rückwärts PCR Primers eingesetzt. Bindung der Sonde an die durch die PCR amplifizierte Ziel-DNA Sequenz, die Komplementär zu der Sondensequenz ist, bricht die Schleifenanordnung auf und separiert dadurch die beiden Farbstoffe, wodurch die FRET-Interferenz aufgehoben und die Fluoreszenz des Reporter-Farbstoffs messbar wird.

Ebenfalls eine direkte Methode der Echtzeit-PCR ist das so genannte "Taqman" Verfahren (C. A. Heid et al., Genom Res. 6, 986 1996). Auch hierbei wird eine FRET-Sonde eingesetzt, die jedoch im Unterschied zu den obengenannten Beacons keine Schleifenanordnung besitzt. Während das Polymerase Enzym den neuen Strang der DNA repliziert, baut die Exo-Nuklease-Aktivität die FRET-Sonde, die an der Ziel DNA gebunden ist, an deren 5' Ende ab, so dass der Reporter-Farbstoff von der Sonde freigesetzt wird. Hierdurch ist der Reporter-Farbstoff nicht mehr räumlich in der Nähe des Quencher-Farbstoffs, so dass seine Fluoreszenz nicht mehr ausgelöscht wird und nun messbar ist. Die Amplifikation der Ziel-DNA und somit die vermehrte Freisetzung des Reporter-Farbstoffs kann dann mittels eines geeigneten optischen Messsystems detektiert werden.

Ein weiteres indirektes Verfahren bestehend aus der Kombination der obengenannten Beacons mit den Primern, wobei ein Primer über eine nicht-amplifizierbare Verbindung mit dem Beacon über dessen 5' Ende verbunden ist. Bei der Amplifikation der Ziel-DNA durch die PCR wird diese Sonde, auch "Scorpion" genannt, mit der Ziel-DNA Sequenz aufgrund der Primer verbunden, selber aber nicht wegen der nicht-amplifizierbaren Verbindung nicht amplifiziert. Bei dem folgenden Denaturierungsschritt kann die zur Ziel-DNA komplementäre Sonden-Sequenz durch Aufbruch der Schleifenanordnung mit der Ziel-DNA Sequenz beim darauf folgenden Abkühlen binden. Durch den Aufbruch der Schleifenanordnung wird die räumliche Nähe der zwei Farbstoffe verhindert und die Fluoreszenz des Reporter-Farbstoffs ist messbar (vgl. oben). Eine Abwandlung dieser "Scorpions" besteht in der Trennung der Farbstoffe in zwei unterschiedliche Oligonukleotide, wodurch die Signalintensität verbessert wird. Die Schleifenkonfiguration ist also durch zwei komplementäre Stränge ersetzt, wobei der Quencher-Farbstoff nun nicht mehr am 3' Ende der Sondensequenz angeordnet ist, sondern auf einem eigenen Strang an dessen 3' Ende, welches dem 5' Ende der Sonde gegenüberliegt. Somit ist die räumliche Nähe der Farbstoffe nur bei der Bindung der komplementären Stränge gegeben. Durch die Denaturierung und folgende Abkühlung trennt sich die eigentliche Sondensequenz von der Quencher-Trägersequenz und erlaubt somit die erwähnte Bindung der Sonde an die Ziel-DNA und die Detektion der Fluoreszenz des Reporter-Farbstoffs.

Für weitere Erläuterungen wird auf Science, Vol 296, Seiten 557 - 558, 19. April 2002 und Journal of Molecular Endocrinology 2002, 29, Seiten 23 - 29 und www.dxsgenotyping.com verwiesen.

Die erfindungsgemäßen Primer dienen also zur Detektion einer antibiotikaspezifischen Nukleotidsequenz aus den zu detektierenden Genen. Die Farbstoffe oder die Sonden mit Farbstoffen markieren wiederum die amplifizierten Antibiotikaresistenz-vermittelnden Gensequenzen zum Nachweis über Fluoreszenz-Messungen. Anhand der Amplifikationsverläufe wird ein Schwellenwert festgelegt, bei dem das Fluoreszenzsignal des Reporter-Farbstoffs deutlich über dem Hintergrundsignal liegt und die Amplifikation der Ziel-DNA unter nicht limitierenden Bedingungen abläuft (linearer Bereich). Aus dem Schnittpunkt der Linie des Schwellenwerts und der Amplifikationskurve ergibt sich ein bestimmter Wert, der ein Maß für die eingesetzte Menge der Ziel-DNA darstellt. Standards mit bekannter Startmenge erlauben eine Kalibrierung über die es dann möglich ist den absoluten Wert der Menge der Ziel-DNA. D. h. des Resistenzgens zu bestimmen.

Die erfindungsgemäßen Primer/Sonden-Systeme sind so ausgewählt, dass sie auf Trägermaterialien immobilisiert werden können. Hierbei handelt es sich üblicherweise um Gold, Glas, Siliziumverbindungen usw., die dem Fachmann bekannt sind.

Ganz besonders sind die erfindungsgemäßen Primer/Sonden-Systeme für die Verwendung auf Biochips geeignet. So können die Primer und Sonden z. B. über Nanospotter auf Träger aufgebracht und immobilisiert werden.

Der erste Farbstoff (C) zum Nachweis der mittels der PCR amplifizierten DNA ist also entweder ein direkter DNA-Farbstoff oder ein Reporter-Farbstoff, der dann zusammen mit dem zweiten Farbstoff (Quencher) eingesetzt wird.

Vorteilhaft ist es daher, wenn der mindestens eine erste Farbstoff (C) bei Bindung an den DNA-Doppelstrang fluoresziert. Dann ist die oben erwähnte erste direkte Methode der Echtzeit-PCR durchführbar. Bei den Farbstoffen handelt es sich um handelsübliche bei Einlagerung in den DNA- oder RNA-Doppelstrang fluoreszierende Farbstoffe, die dem Fachmann bekannt sind. Somit können die bei der PCR neu entstehenden Doppelstränge nachgewiesen werden. Besonders häufig wird der Farbstoff SYBR Grün eingesetzt.

Wenn zusätzlich eine Sonden-Nukleotidsequenz (D) ausgewählt aus der Gruppe der Sequenzen bestehend aus den SEQ # 9 - 12 eingesetzt wird, kann eine der anderen direkten oder indirekten Methoden der Echtzeit-PCR mit den erfindungsgemäßen Primern durchgeführt werden. Auch die Sequenzen der Sonden sind in Fig. 1 angegeben.

Vorteilhaft ist es dazu, wenn der mindestens eine erste Farbstoff (C) mit der Sonden-Nukleotidsequenz (D), insbesondere über deren 5' Ende verbunden ist.

Falls eine der Primer-Nukleotidsequenzen (A, B) mit der Sonden-Nukleotidsequenz (D) über deren 3' Ende verbunden ist und das 3' Ende ferner über eine mittels PCR nicht-amplifizierbare Verbindung (E) mit der Primer-Nukleotidsequenz (A, B) verbunden ist, kann als Methode die besonders viel versprechende indirekte Methode der "Scorpione" eingesetzt werden, die sich insbesondere durch ihre schnelle unimolekulare Reaktion auszeichnet.

Ferner ist ein zweiter Farbstoff (F) nötig, der direkt mit der Sonden-Nukleotidsequenz (D) verbunden sein kann und die Fluoreszenz des ersten Farbstoffs (C) bei räumlicher Nähe durch die sogenannte FRET (Fluoreszenz Resonanter Energie Transfer) löscht.

Dieser kann vorteilhafterweise mit der Sonden-Nukleotidsequenz (D) über deren 3' Ende verbunden sein. Dann liegt ein einteiliger "Scorpion" vor. Die Sonden-Nukleotidsequenz (D) wird dann mittels komplementären Sequenzen an ihren 5' und 3' Enden in einer Schleifenkonfiguration (Hairpin Loop) gehalten. Somit liegen der erste Farbstoff (Reporter) und der zweite Farbstoff (Quenscher) räumlich eng bei einander und es tritt keine Fluoreszenz auf (vgl. oben).

Alternativ ist es möglich, dass der mindestens eine zweite Farbstoff (F) mit einer der Sonden-Nukleotidsequenz (D) komplementären Sequenz (G) über deren 3' Ende verbunden ist. Dann liegt ein zweiteiliger "Scorpion" vor (vgl. oben).

Wird bei einer Schleifenkonfiguration der Sonden-Nukleotidsequenz (D) auf die Verbindung mit der Primer-Nukleotidsequenz verzichtet und die Primer - wie üblich einzeln hinzugesetzt - dann liegt ein so genannter "Beacon" vor, der ebenfalls nur im gebundenen Zustand fluoresziert (vgl. oben).

Die Erfindung umfasst ferner ein System zur Verwendung in dem oben beschriebenen Verfahren zur Detektion von Antibiotikaresistenz-vermittelnden Genen in Mikroorganismen mittels der Echtzeit-PCR umfassend:
- eine erste Primer-Nukleotidsequenz (A) ausgewählt aus der Gruppe der Sequenzen bestehend aus den SEQ # 1 - 4,
- eine zweite Primer-Nukleotidsequenz (B) ausgewählt aus der Gruppe der Sequenzen bestehend aus den SEQ # 5 - 8, wobei die Sequenzen 1 und 5, 2 und 6, 3 und 7, 4 und 8 als Primerpaare eingesetzt sind, und
- mindestens einen ersten Farbstoff (C) zum Nachweis der mittels der PCR amplifizierten DNA,
- ggf. eine Sonden-Nukleotidsequenz (D) ausgewählt aus der Gruppe der Sequenzen bestehend aus den SEQ # 9 - 12,
- ggf. einen zweiten Farbstoff (F), der die Fluoreszenz des ersten Farbstoffs (C) bei räumlicher Nähe löscht. Nachfolgend wird die Erfindung beispielhaft beschrieben.

### Referenzbakterienstämme

*Enterococcus faecium* B7641 *van*A^{r} diente als Referenzstamm für das *van*A Gen. Die Stämme *Staphylococcus aureus* A1 *mec*A^{r} und *Enterobacter cloacae* A10*amp*C^{r} wurden taxonomisch sowohl durch Sequenzierung als auch über ihre Resistenzgene identifiziert und jeweils als Referenz eingesetzt. *Pseudomonas aeruginosa* 15 wurde isoliert und mit Hilfe des Kits API 20NE (bioMerieux, Nürtlingen Deutschland) ebenfalls taxonomisch identifiziert und als Referenz für bla_{VIM}-2 eingesetzt. Der Stamm *Pseudomonas aeruginosa* VR 143/97 diente als Referenz für das Gen bla_{VIM}-1.

Als antibiotoka-empfindliche Kontrollstämme dienten *Staphylococcus aureus* ssp. *aureus* DSM 20231 mecA^{S}, *Enterococcus faecium* DSM 20477 vanA^{S}, für sensitive Enterobacteriaceae *Escherichia coli* DSM 1103 ampC^{S} und *Pseudomonas aeruginosa* 22 VIM^{S}.

### Probenentnahme und Vorbereitung

Wasserproben (500 ml) wurden aus dem Einlauf, Klärschlamm und dem Abwasser öffentlicher Kläranlagen und dem Abwasser von Krankenhäusern (klinisches Abwasser) zur Kultivierung und DNA Extraktion entnommen.

Enterokokken wurden durch Kultivierung bei 37 °C über 24 h in Azid-Dextrosebrühe (Oxoid, Basingstoke, England) angereichert. Vancomycin-resistente Isolate wurden durch Selektion auf Kanamycin-Esculin-Azid-Agar (Merck KG aA, Darmstadt, Deutschland) beinhaltend 32 µg Vancomycin pro mL gemäß NCCLS gewonnen. Aufgrund des großen Vorkommens von *Enterobacteriaceae* im Abwasser wurden Isolate durch Kultivierung auf Chromocult Agar (Merck KG aA, Darmstadt, Deutschland) mit 32 µg/mL Ceftazidim als Antibiotikum zur Resistenzselektion ohne vorherige Anreicherung gewonnen.

Referenzstämme von *Enterobacter cloacae* und *Enterococcus faecium* wurden suspendiert und in einer abnehmenden Reihe in PBS (137 mM NaCl, 7,25 mM Na₂HPO4, 0,2 mM KH₂PO4, 2,7 mM KCL, pH 7,4) verdünnt und zur Quantifizierung mittels "plate count" auf R2A Agar (Difco) oder Slanetz-Bartley Agar (Merck) kultiviert.

### Primer/Sonden Desiqn

Die Sequenzen der Resistenzgene wurden aus der NCBI Datenbank entnommen:

| Gen | Nummer |
|---|---|
| *Enterobacter cloacae amp*C | AF411145 |
| *Pseudomonas aeruginosa bla*_{VIM} | Y18050 |
| *Staphylococcus aureus mec*A | E09771 |
| *Enterococcus faecium* Plasmid pIP816 *van*A | X56895 |

Mittels der Software Primer Express der Firma Applied Biosystems wurden die Primer- und Sondensequenzen zur Verwendung in einem standardisierten TaqMan Amplifikationsprotokoll entwickelt. Alle Primer und fluorogenen Sonden wurden von der Firma Applera (Darmstadt, Deutschland) synthetisiert.

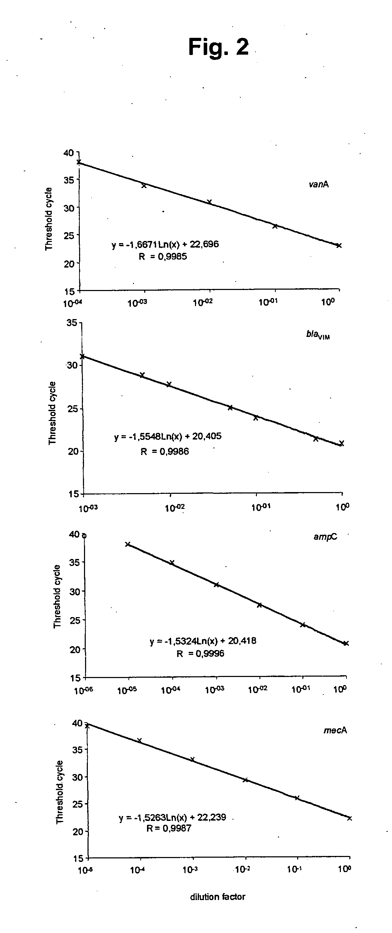

Die Spezifität der Primer und Sonden wurde durch Sequenzvergleich mittels BLAST Verfahren mit den NCBI Einträgen ermittelt. Auf eine Kreuzreaktion wurde mit den entsprechenden antibiotika-sensitiven Kontrollstämmen getestet. Die erfindungsgemäßen Primer/Sonden Systeme wurden ferner mittels dreifach durchgeführter PCR anhand von seriell verdünnten Referenzstämmen getestet und Cₜ Kalibriergeraden aufgestellt (Fig. 2).

### PCR

Von der Firma Applied Biosystems wird ein für den Ansatz von quantitativen PCR-Assays optimierter universal Master Mix (uMM) verwendet, der dNTPs, AmpliTaq Gold® DNA Polymerase, AmpErase® UNG (Uracil-N-Glykosidase), MgCl₂ und Pufferkomponenten sowie den fluorogenen Farbstoff ROX als passive Referenz enthält, so dass Pipettierfehler von der Auswertungssoftware automatisch korrigiert werden können.

Die bei der TaqMan-PCR eingesetzte Polymerase AmpliTaq Gold (Applied Biosystems) ist eine rekombinante Form der AmpliTaq DNA Polymerase und wurde erst durch eine 9 bis 12 minütigen Inkubationsschritt bei 90 °C irreversibel aktiviert.

Zur Optimierung der Sondenhybridisierung wurde unter Standardbedingungen eine Zweischritt PCR durchgeführt. Dies wird ermöglicht durch die signifikante Aktivität der AmpliTaq Gold Polymerase bei Temperaturen von ≥ 55 °C und eine Auswahl von Primern mit einer einheitlichen Annealing-Temperatur um 60 °C. Dadurch kann ein vereinheitlichtes Zweischritt PCR-Protokoll durchgeführt werden, bei dem für die Amplifikation nur ein 95 °C-Schritt zur Denaturierung und ein 60 °C-Schritt für Annealing und Extension benötigt werden.

Zum Schutz vor Kontaminationen wurde mit der AmpErase UNG zunächst ein zweiminütiger Inkubationsschritt bei 50 °C durchgeführt.

Zur Amplifikation mittels der Echtzeit PCR wurde ein ABI 7000 oder 7700 Sequenz Detektor System (Applied Biosystems) verwendet.

Zur Durchführung der PCR wurden 10 µL eines Templats (zu untersuchende Probe) in 50µL Reaktionsvolumina amplifiziert, die 300 nM jedes Primers, 200 nM einer FAM/TAMARA-markierten Sonde und 25 µL 2-fach TaqMan universal Master Mix und 7 µL Wasser enthielten einem standard TaqMan Temperaturprofil (2 min bei 50 °C, 10 min bei 95 °C und 40 Zyklen zu je 15 s bei 95 °C, 1 min bei 60 °C) unterworfen.

### Taxonomische und Resistenzgen-Identifikation durch Sequenzierung

Stämme wurden taxonomisch durch partielle Sequenzierung der 16S rDNA identifiziert. Die Universalprimer 27F (5'-AGAGTTTGATCMTGGCTCAG-3', SEQ ID 13) und 517R (5'-ATTACCGCGGCTGCTGG-3', SEQ ID 14) (Muyzer et al., Appl. Environ. Microbiol. 59(3), 695, 1993; Kilb et al., Acta Hydrochim. Hydrobiol. 26(6), 349, 1998) wurden zur Erzeugung eines 526 Basenpaare umfassenden Amplikons der Stellen 8 bis 534 der 16S rDNA von E. coli (Brosius et al., J. Mol. Biol. 147, 107, 1981) verwendet. Ein PCR Profil mit 35 Zyklen aus 94 °C für 30 s, 49 °C für 30 s und 72 °C für 1 min nach Aktivierung der HotStart Taq Polymerase (Qiagen, Hilden) bei 95 °C für 15 min und ein Schlussextensionszyklus bei 72 °C für 7 min wurden angewandt.

Der 27F Primer wurde auch für die Sequenzierungsreaktion eingesetzt. Zur Überprüfung auf das Vorhandensein des Resistenzgens vanA wurde ein bestimmtes PCR Produkt mittels der Primer vanA1 (5'-TCTGCAATAGAGATAGCCGC-3', SEQ ID 15) und vanA2 (5'-GGAGTAGCTATCCCAGCATT-3', SEQ ID 16) amplifiziert (Klein et al., Appl. Environ. Microbiol. 64, 1825, 1998). Anschließend wurde der Primer *vanA1* als Primer für die Sequenzierung eingesetzt. Das Resistenzgen *ampC* wurde gemäß Schwartz et al. (FEMS Microbiol. Ecol. 43(3), 325, 2003) mittels der Primer ampC-For (5'-TTCTATCAAMACTGGCARCC-3', SEQ ID 17) und ampC-Rev (5'-CCYGTTTTATGTACCCAYGA-3', SEQ ID 18) amplifiziert. Das Resistenzgen *mecA* wurde gemäß Murakami et al. J. Clin. Microbiol. 29, 2240, 1991) mittels der Primer mecA1 (5'-AAAATCGATGGTAAAGGTTGGC-3', SEQ ID 19) und mecA2 (5'-AGTTCTGCAGTACCGGATTTGC-3', SEQ ID 20) amplifiziert. Alle Amplifizierungen wurden mit einem GeneAmp PCR System 9700 der Fa. Applied Biosystems durchgeführt.

Die Sequenzierungen der PCR Produkte wurden mit der Sanger Methode (Sambrook et al., Molecular cloning: a laboratory manual, Harbor Laboratory Press, Cold Spring Harbor, New York 2001) mittels des BigDye Terminator Cycle Sequencing Ready Reaction Chemistry Kits der Fa. Applied Biosystems durchgeführt. Die Sequenzirungsreaktion wurde mit einem Denaturierungsschritt bei 95 °C für 5 min begonnen, gefolgt von 25 Zyklen bei 55 °C für jeweils 5 s und beendet mit einer Extensionsreaktion bei 60 °C für 1 min (GeneAmp PCR System 9700 der Fa. Applied Biosystems). Die erhaltenen Fragmente wurden mit einem ABI Prism 310 Genetic Analyser der Fa. Appl. Biosystems getrennt und analysiert. Mit den so erhaltenen DNA Sequenzen wurden BLAST DNA Homologie suchen in der NCBI Datenbank durchgeführt.

### Ergebnisse

Das Abwasser von fünf öffentlichen Kläranlagen wurde auf das Vorkommen von antibiotikaresistenten Bakterien untersucht. Vancomycin-resistente Enterokokken und β-Lactam-resistente *Enterobacteriaceae* wurden nach spezifischer Anreicherung aus allen Abwasser Proben isoliert.

Die Isolate wurden zunächst biochemisch mittels der Testkits rapid ID32 strep und API 20E (BioMerieux, Nürtingen, Deutschland) als *Enterococcus faecium* und *Enterobacter cloacae* identifiziert. Diese Ergebnisse wurden mittels der Sequenzanalyse basierend auf der 16S rDNA bestätigt. Die Enterokokken Stämme aus EF1 bis EF4 zeigten 99 bis 100 % Homologie mit *Enterococcus faecium* und die isolierten *Enterobacteriaceae* EB4, EB86, EB101 und EB102 zeigten 98 bis 100% Homologie mit *Enterobacter cloacae*.

Vorausgehende Untersuchungen (Schwartz et al., vgl. oben) und Kultivierungsexperimente zeigten, dass keine Staphylokokken aus öffentlichen Abwasserproben isoliert werden konnten. Daher wurden klinische Isolate S1 bis S4, bei welchen es sich um Methicillin-resistente Staphylokokken aus Patienten der Universität Heidelberg handelt, für die real-time PCR Experimente eingesetzt. Ihre taxonomische Identität als *Staphylococcus aureus* wurde durch eine 99-% Homologie mit den NCBI Datenbank Einträgen bestätigt.

Mit den oben beschriebenen resistenten Bakterien wurden spezifische PCR Experimente zur Amplifikation der Resistenzgene *vanA*, *ampC* und *mecA* durchgeführt. Sowohl die PCR Ergebnisse und nachfolgende Sequenzierung zeigten, dass die Resistenz der Enterokokken durch das *vanA* Gen, die Resistenz der *Enterobacter cloacae* und *E. coli* durch das *ampC* Gen und die Methicillin-Resistenz der Staphylokokken durch das *mecA* Resistenzgen hervorgerufen werden. Alle resistenten Stämme zeigten eine Homologie von 99 bis 100% mit den NCBI Datenbankeinträgen.

Die entwickelten Primer/Sonden Systeme sind in Tabelle 1 dargestellt.

Die Cₜ-Werte (Tabelle 2) wurden durch Amplifikation und Auftragung der Ergebnisse von Proben seriell verdünnter DNA der Referenzstämme ermittelt. Figur 1 zeigt dies beispielhaft für *Enterobacter cloacae amp*C. Anschließend wurden diese Daten zur Erstellung der in Figur 2 dargestellten Eichgeraden verwendet. Bei einer halblogarithmischen Auftragung stellen die linearen Datenbereiche die quantifizierbaren Messbereiche der Primer/Sonden Systeme dar.

Um falsch-positive Ergebnisse zu vermeiden wurden in allen PCR Assays Kontrollversuche ohne Template (NTC, no template control), d. h. ohne bakterieller DNA, und ohne komplementärer Sequenz (NAC, no amplification control,) in der bakteriellen DNA durchgeführt (vgl. Tabelle 2).

35 Abwasserproben von fünf Kläranlagen und zwei Krankenhausabwasserproben wurden auf das Vorkommen der Resistenzgene *vanA*, *ampC* und *mecA* hin untersucht. Aus 30 bis 50 ml Probenvolumen konnten mittels geeigneter kommerziell erhältlicher Extraktionskits zwischen 9 und 100 µg Gesamt-DNA extrahiert werden. Diese DNA konnte für die genannten TaqMan Systeme in der Real-Time PCR eingesetzt werden. In 78 % der Proben konnte *ampC* gefunden werden. Die dazugehörigen Cₜ-Werte sind in Tabelle 1 aufgeführt. Das Gen *vanA* trat mit 22 % deutlich seltener auf, während das Gen *mecA* nicht im Abwasser in nachweisbaren Konzentration auftrat.

Mit Kultivierverfahren konnte jedoch aus den entsprechenden Umweltkompartimenten neben resistenten *Enterococcus faecium* und *Enterobacter cloacae* auch *Staphylococcus aureus* isoliert werden. Der Nachweis der entsprechenden Resistenzgene einiger dieser Isolate mit den dazugehörigen Ct-Werten ist ebenfalls der Tabelle 3 zu entnehmen.

Die Durchführung von Taqman-PCR an den Referenzstämmen zeigte, dass das Taqman-System vim1 den Nachweis des Gens bla_{VIM}-2 ermöglicht, während bla_{VIM}-1 nicht die detektiert wird (Tabelle 4).

Für Untersuchungen zum Auftreten des bla_{VIM} Gens wurden Abwasserproben mit unterschiedlichen klinischen Anteilen untersucht. Dabei konnte nach DNA-Extraktion in einer Abwasserprobe ohne vorherige Anreicherung der Zielorganismen das Vorkommen von bla_{VIM}-2 nachgewiesen werden (vgl. Tabelle 4).

**Tabelle 1.**

| Resistenzgen | Antibiotikum | Zielorganismus | TaqMan System | Primer-Sequenzen | Sonden-Sequenz |
|---|---|---|---|---|---|
| *bla*_{*VIM*} | Imipenem | *Pseudomonas aeruginosa* | vim1 | vim1FP: 5 '-cctccattgagcggattca-3' (SEQ #1) | vim1: 5'-caacactacccggaagcacagtt cgtc-3' (SEQ #9) |
| | | | | vim1RP 5 -'gccgtgccccggaa-3' (SEQ #5) | |
| *ampC* | Ampicillin | *Enterobactercloacae* | ampC | Lak1 FP: 5'-gggaatgctggatgcacaa-3' (SEQ #2) | P-Lak1: '5'-cctatggcgtgaaaaccaacgtg ca-3' (SEQ #10) |
| | | | | LakA1RP: 5'-catgacccagttcgccatatc-3' (SEQ #6) | |
| *mecA* | Methicilin | CNS, *Staphylococcus aureus* (MRSA) | mecA1 | mecA1 FP 5 '-cgcaacgttcaatttaattttgttaa -3' (SEQ #3) | mecA1: 5'-aatgacgctatgatcccaatctaacttccaca-3' (SEQ #11) |
| | | | | mecA1 RP: 5 '-tggtctttctgcattcctgga-3' (SEQ #7) | |
| vanA | Vancomycin | *Enterococcus faecium* | vana3 | vanA3FP:5'-ctgtgaggtcggttgtgcg-3' (SEQ #4) | vanA3: 5'-caactaacgcggcactgtttccca at-3' (SEQ #12) |
| | | | | vanA3RP: 5 '-tttggtccacdcgcca-3' (SEQ #8) | |

**Tabelle 2.**

| TaqMan System | Schwellenwert (ΔRn) | Ctₘᵢₙ | Ctₘₐₓ | Ct sensitiver Referenzbakterien( NAC) | Ct_{NTC} |
|---|---|---|---|---|---|
| *vim1* | 0,22 | 20,7 | 31,1 | > 40 | > 40 |
| *ampC* | 0,19 | 20,6 | 38,0 | > 40 | > 40 |
| *mecA1* | 0,20 | 22,1 | 39,3 | > 40 | > 40 |
| *vana3* | 0,29 | 22,8 | 38,2 | > 40 | > 40 |
| NTC: no template control | | | | | |
| NAC: no amplification comtrol | | | | | |

**Tabelle 3.**

| | **Art der Probe** | **Probe** | **C**_{**t-**}**WERT** |
|---|---|---|---|
| **vanA** | Referenz | *Enterococcus faecium* B7641 | 16,0 |
| | sensitive Referenz | *Enterococcus faecium* DSM 20477 | > 40 |
| | resistente Isolate | EF 1 | 15,6 |
| | | EF 2 | 16,0 |
| | | EF 3 | 18,0 |
| | | EF 4 | 16,5 |
| | Gesamt-DNA Abwasser | ww2 | 31,9 |
| | | ww3 | 34,7 |
| | | ww6 | 33,7 |
| | | ww7 | 27,6 |
| | | 15 Proben | > 40 |
| **ampC** | Referenz | *Enterobacter cloacae* P A10 | 15,4 |
| | sensitive Referenz | *E. coli* DSM 1103 | > 40 |
| | resistente Isolate | EB 4 | 19,4 |
| | | EB 86 | 18,2 |
| | | EB 101 | 18,4 |
| | | EB 102 | 18,9 |
| | Gesamt-DNA Abwasser | ww8 | > 40 |
| | | ww9 | > 40 |
| | | ww10 | 34,5 |
| | | ww11 | 35,3 |
| | | ww12 | 27,3 |
| | | ww13 | 29,1 |
| | | ww14 | 28,1 |
| | | ww15 | 31,4 |
| | | ww16 | 29,4 |
| **mecA** | Referenz | *S. aureus* A1 | 20,5 |
| | sensitive Referenz | *S. aureus* DSM 20231 | > 40 |
| | resistente Isolate | S1 | 21,3 |
| | | S2 | 19,7 |
| | | S3 | 19,7 |
| | | S4 | 19,4 |
| | Gesamt-DNA Abwasser | ww8 | > 40 |
| | | ww9 | 38,6 |
| | | ww17 | > 40 |
| | | ww18 | > 40 |
| | | ww19 | > 40 |
| | | ww20 | > 40 |
| | | ww21 | 38,6 |

**Tabelle 4.**

| | **Art der Probe** | **Probe** | **C**_{**t-**}**WERT** |
|---|---|---|---|
| **bla**_{**VIM**} | Referenz bla_{VIM}-2 | Ps. aeruginosa 15 | 20,7 |
| | Referenz bla_{VIM}-1 | Ps. aeruginosa VR 143/97VR | > 40 |
| | sensitive Referenz | Ps. aeruginosa 22 | > 40 |
| | resistente Abwasserisolate | Ps. aerug. 1 | 22,2 |
| | | Ps. aerug. 9 | 22,7 |
| | | Ps. aerug. 15 | 23,1 |
| | | Ps. aerug. 16 | 22,4 |
| | | Ps. aerug. 23 | 23,0 |
| | | Ps. aerug. 39 | 18,5 |
| | | Ps. aerug. 49 | 19,3 |
| | | Ps. aerug. 56 | 17,1 |
| | | Ps. aerug. 72 | 17,9 |
| | | Ps. aerug. 76 | 17,3 |
| | resistente Zuwasserisolate | Ps. aerug. 81 | 20,8 |
| | | Ps. aerug. 83 | 18,3 |
| | Gesamt-DNA Abwasser | wwB1 | > 40 |
| | | wwB2 | > 40 |
| | | wwB3 | 35,4 |
| | | wwB4 | > 40 |
| | | wwB5 | > 40 |

## Patentansprüche

1. Oligonukleotid umfassend eine Nukleotidsequenz ausgewählt aus der Gruppe der Sequenzen bestehend aus den SEQ # 1 - 8.

2. Oligonukleotid nach Anspruch 1, **dadurch gekennzeichnet, dass** es als Primer für die PCR verwendbar ist.

3. Oligonukleotid umfassend eine Nukleotidsequenz ausgewählt aus der Gruppe der Sequenzen bestehend aus den SEQ # 9 - 12.

4. Oligonukleotid nach Anspruch 2, **dadurch gekennzeichnet, dass** es als Sonde für die Echtzeit-PCR verwendbar ist.

5. Verfahren zur Detektion von Antibiotikaresistenz-vermittelnden Genen in Mikroorganismen mittels der Echtzeit-PCR umfassend:
- Einsatz mindestens eines ersten Oligonukleotids (A) gemäß Anspruch 1 oder 2 als Primer,
und
- Einsatz mindestens eines ersten Farbstoffs (C) zum Nachweis der mittels der PCR amplifizierten DNA.

6. Verfahren nach Anspruch 5, wobei
- die erste Primer-Nukleotidsequenz (A), aus der Gruppe der Sequenzen bestehend aus den SEQ # 1 - 4 ausgewählt wird,
- eine zweite Primer-Nukleotidsequenz (B), aus der Gruppe der Sequenzen bestehend aus den SEQ # 5 - 8 ausgewählt wird, und
- die Sequenzen SEQ # 1 und 5, 2 und 6, 3 und 7, 4 und 8 als Primerpaare eingesetzt werden.

7. Verfahren nach Anspruch 5 oder 6, **dadurch gekennzeichnet, dass** der mindestens eine erste Farbstoff (C) bei Bindung an den DNA-Doppelstrang fluoresziert.

8. Verfahren nach einem der Ansprüche 5 bis 7, **dadurch gekennzeichnet, dass** ein Oligonukleotid (D) gemäß Anspruch 3 oder 4 als Sonde eingesetzt wird.

9. Verfahren nach Anspruch 8, **dadurch gekennzeichnet, dass** der mindestens eine erste Farbstoff (C) mit der Sonden-Nukleotidsequenz (D), insbesondere über deren 5' Ende verbunden ist.

10. Verfahren nach Anspruch 8 oder 9, **dadurch gekennzeichnet, dass** eine der Primer-Nukleotidsequenzen (A, B) mit der Sonden-Nukleotidsequenz (D) über deren 3' Ende verbunden ist.

11. Verfahren nach einem der Ansprüche 8 bis 10, **dadurch gekennzeichnet, dass** das 3' Ende der Sonden-Nukleotidsequenz (D) über eine mittels PCR nicht-amplifizierbare Verbindung (E) mit einer Primer-Nukleotidsequenz (A, B) verbunden ist.

12. Verfahren nach einem der Ansprüche 5 bis 11, **dadurch gekennzeichnet, dass** ein zweiter Farbstoff (F) mit der Sonden-Nukleotidsequenz (D) verbunden ist, der die Fluoreszenz des ersten Farbstoffs (C) bei räumlicher Nähe löscht.

13. Verfahren nach Anspruch 12, **dadurch gekennzeichnet, dass** der mindestens eine zweite Farbstoff (F) mit der Sonden-Nukleotidsequenz (D) über deren 3' Ende verbunden ist.

14. Verfahren nach einem der Ansprüche 8 bis 13, **dadurch gekennzeichnet, dass** die Sonden-Nukleotidsequenz (D) mittels komplementären Sequenzen an ihren 5' und 3' Enden in einer Schleifenkonfiguration gehalten wird.

15. Verfahren nach einem der Ansprüche 8 bis 12, **dadurch gekennzeichnet, dass** der mindestens eine zweite Farbstoff (F) mit einer der Sonden-Nukleotidsequenz (D) komplementären Sequenz (G) über deren 3' Ende verbunden ist.

16. Verfahren nach einem der Ansprüche 5 bis 15, **dadurch gekennzeichnet, dass** die Antibiotika aus der Gruppe bestehend aus Imipinem, Ampillicin, Methicillin, Vancomycin ausgewählt sind.

17. Verfahren nach einem der Ansprüche 5 bis 16, **dadurch gekennzeichnet, dass** die Antibiotikaresistenz-vermittelnden Gene aus der Gruppe bestehend aus bla_{VIM}, ampC, mecA, vanA ausgewählt sind.

18. Verfahren nach einem der Ansprüche 5 bis 17 **dadurch gekennzeichnet, dass** die Mikroorganismen aus der Gruppe bestehend aus Pseudomonas aeruginosa, Enterobacter cloacae, Staphylococcus aureus, Enterococcus faecium ausgewählt sind.

19. Verfahren nach einem der Ansprüche 5 bis 18, **dadurch gekennzeichnet, dass** die Nukleotidsequenzen auf einem Trägermaterial, insbesondere auf einem Biochip immobilisiert sind.

20. System zur Verwendung in einem Verfahren nach einem der Ansprüche 5 bis 19 umfassend:
- eine erste Primer-Nukleotidsequenz (A) ausgewählt aus der Gruppe der Sequenzen bestehend aus den SEQ # 1 - 4,
- eine zweite Primer-Nukleotidsequenz (B) ausgewählt aus der Gruppe der Sequenzen bestehend aus den SEQ # 5 - 8, wobei die Sequenzen 1 und 5, 2 und 6, 3 und 7, 4 und 8 als Primerpaare eingesetzt sind, und
- mindestens einen ersten Farbstoff (C) zum Nachweis der mittels der PCR amplifizierten DNA,
- ggf. eine Sonden-Nukleotidsequenz (D) ausgewählt aus der Gruppe der Sequenzen bestehend aus den SEQ # 9 - 12,
- ggf. einen zweiten Farbstoff (F), der die Fluoreszenz des ersten Farbstoffs (C) bei räumlicher Nähe löscht.

21. Verwendung eines Systems nach Anspruch 20 oder eines Oligonukleotids nach einem der Ansprüche 1 bis 4 zur Immobilisierung auf einem Trägermaterial, insbesondere einem Biochip.

22. Verwendung eines mit einem System nach Anspruch 20 oder einem Oligonukleotid nach einem der Ansprüche 1 bis 4 versehenen Trägermaterials, insbesondere eines Biochips zur Detektion von Antibiotikaresistenz-vermittelnden Genen in Mikroorganismen mittels der PCR, insbesondere nach einem der Verfahren nach einem der Ansprüche 5 bis 19.
